Europäisches Patentamt

European Patent Office

Office européen des brevets

(19)

(11) Publication number: **0 266 840**

**A1**

## (12) EUROPEAN PATENT APPLICATION

(21) Application number: 87202110.0

(22) Date of filing: 02.11.87

(51) Int. Cl.⁴: **C07C 126/02 , C07C 126/08**

(30) Priority: 03.11.86 NL 8602770

(43) Date of publication of application:
**11.05.88 Bulletin 88/19**

(84) Designated Contracting States:
**AT BE DE ES FR GB GR IT NL SE**

(71) Applicant: **STAMICARBON B.V.**
**Mijnweg 1**
**NL-6167 AC Geleen(NL)**

(72) Inventor: **Jonckers, Kees**
**Illikhoven 13**
**NL-6116 AK Susteren(NL)**
Inventor: **Burks, Henk Christiaan**
**Drossaertweide 20**
**NL-6438 HV Schinnen(NL)**

(74) Representative: **Hoogstraten, Willem Cornelis**
**Roeland et al**
**OCTROOIBUREAU DSM Postbus 9**
**NL-6160 MA Geleen(NL)**

(54) Process for preparing urea.

(57) Process for preparing urea in which,
-at a pressure of 125-350 bar and the corresponding temperature, a urea synthesis solution containing carbamate is formed from carbon dioxide and an excess of ammonia,
-in a first decomposition step part of the carbamate is decomposed, with supply of heat and in countercurrent contact with a stripping gas, and the resulting gas phase is in part condensed in a first con-densation zone,
-in at least two further decomposition steps a further part of the carbamate still present is decomposed and the gas mixture formed is separated off, in the first of the further decomposition steps a pressure of 4-40 bar being maintained,
-the remaining urea-containing solution is processed by evaporation to form a concentrated urea solution, the heat required for evaporation being obtained at least in part by condensation of the gas mixture obtained in the first of the further decomposition steps.

The gas mixture used in the first evaporation step is formed by the gas phase resulting from the heat exchange between the part of the stripped urea synthesis solution expanded to a pressure of 4-40 bar and the condensing gas mixture in the first condensation zone.

# PROCESS FOR PREPARING UREA

The invention relates to a process for preparing urea from ammonia and carbon dioxide.

If ammonia and carbon dioxide are fed into a synthesis zone under a suitable pressure (for instance 125-350 atm) and at a suitable temperature (for instance 170-250¤C), ammonium carbamate is first formed, according to the reaction:

$$2 NH_3 + CO_2 \rightarrow H_2N\text{-}CO\text{-}ONH_4$$

Subsequently, urea is formed from the resulting ammonium carbamate by dehydration according to the reversible reaction:

$$H_2N\text{-}CO\text{-}ONH_4 \rightleftharpoons H_2N\text{-}CO\text{-}NH_2 + H_2O$$

The degree to which the conversion into urea proceeds depends on, among other factors, the temperature and the excess of ammonia that is used. The resulting reaction product is a solution consisting substantially of urea, water, ammonium carbamate and free ammonia. The ammonium carbamate and the ammonia must be removed from the solution and are in most cases returned to the synthesis zone. The synthesis zone may consist of separate zones for the formation of carbamate and urea. These zones, however, may also be combined in one apparatus.

A process frequently applied for preparing urea is described in European Chemical News, Urea Supplement, of 17th January, 1969, pages 17-20. In that process the urea synthesis solution formed in the synthesis zone at high pressure and temperature is stripped at synthesis pressure by contacting the solution countercurrently with gaseous carbon dioxide while supplying heat, so that the larger part of the carbamate present in the solution is decomposed into ammonia and carbon dioxide, which decomposition products are removed from the solution in gaseous form and discharged together with a small amount of water vapour and the carbon dioxide used for the stripping. Besides with carbon dioxide, as described in that publication, such stripping can also be done with gaseous ammonia, an inert gas or with a mixture of at least two of the gases mentioned. The heat required for stripping is obtained by condensation of high pressure steam of 15-25 bar on the shell side of the tubes of the vertical heat exchanger in which stripping is effected. In a condensation zone maintained at synthesis pressure the gas mixture obtained in the stripping process is for the greater part condensed and absorbed in an aqueous solution originating from the further treatment of the urea-containing solution, upon which the aqueous carbamate solution thus formed as well as the non-condensed part of the gas mixture are passed to the synthesis zone for the formation of urea. Here the heat required for the conversion of carbamate into urea is obtained by further condensation of the gas mixture.

The stripped urea synthesis solution is subsequently expanded to a low pressure of, for instance, 2-6 bar and heated by means of steam in order to remove from the solution ammonia and carbon dioxide still present in the stripped urea solution partly in the form of carbamate. The gas mixture resulting from these operations, which also contains water vapour, is condensed and absorbed in an aqueous solution in a condensation zone operating at low pressure and the resulting diluted carbamate solution is pumped back to the high pressure section of the urea synthesis and eventually passed into the synthesis zone. The remaining urea-containing solution is subjected to further expansion and upgraded to form a urea solution or melt that may be further processed to form solid urea. To this end the aqueous urea solution is usually evaporated in two evaporation steps and the resulting urea melt is processed to form granules or the urea solution is crystallized. The gases obtained in the evaporation or crystallization, which, in addition to water vapour, contain, among other substances, ammonia, carbon dioxide and entrained fine urea droplets, are condensed, during which process so-called process condensate is formed. Part of the process condensate is used as absorbent for the gas mixture in the low pressure condensation zone. The remaining part can be treated with high pressure steam for the purpose of decomposing urea contained therein into ammonia and carbon dioxide and recovering these decomposition products along with the ammonia and carbon dioxide already present as such.

It has already been proposed to subject the gas mixture obtained in stripping to two-step condensation in such a process (see US-A 4,354,040). In the first step heat exchange takes place with a liquid phase consisting substantially of an aqueous urea solution which also contains biuret, ammonia and carbamate. This liquid phase is formed by at least part of the liquid obtained in a gas-liquid separator to which the stripped urea synthesis solution is fed upon expansion to a pressure of approx. 18 bar. In the second step condensation takes place with the help of water, low pressure steam being formed simultaneously.

It has also already been proposed to use, when evaporating urea solutions in two steps, in the first step the heat released in the condensation of gas mixtures containing ammonia and carbon dioxide which are obtained at a sufficiently high pressure level in the urea preparation process, after increasing the dew point of the mixture to be condensed to such an extent that almost the entire gas mixture condenses in the heat exchange (see the Netherlands patent application 8303888). It was also proposed to supply, in addition to the heat released in the condensation of the gas mixture, an amount of heat obtained by condensation of low pressure steam, so as to be able to cover the total heat requirement of the first evaporation step.

The aim of the invention is to provide a process for preparing urea in which the heat generated in the condensation of the gas mixture obtained in the stripping process is used as economically as possible, particularly in the evaporation of the urea solution ultimately obtained, use beingd, use being also made of the heat developed during the further processing of the stripped urea synthesis solution. This is achieved according to the invention by dividing the gas mixture obtained in the stripping process between two condensation zones maintained at synthesis pressure, the condensation heat generated in one of these zones being used to evaporate the urea solution.

The invention consequently relates to a process for preparing urea in which,
-in a synthesis zone, at a pressure of 125-350 bar and the corresponding temperature, a urea synthesis solution containing carbamate and free ammonia is formed from carbon dioxide and an excess of ammonia,
-in a first decomposition step part of the carbamate is decomposed, at synthesis pressure or lower, with supply of heat and in countercurrent contact with a stripping gas, and the resulting gas mixture is partly condensed in a first condensation zone and the condensate and the non-condensed part of the gas mixture are returned to the synthesis zone,
-in at least two further decomposition steps a further part of the carbamate still present is decomposed and the gas mixture formed is separated off, in the first of the further decomposition steps a pressure of 4-40 bar being maintained and heat being supplied,
-the remaining urea-containing solution is processed by evaporation to form a concentrated urea solution, the heat required for evaporation being, at least in part, obtained by condensation, at the pressure of 4-40 bar, of the gas mixture obtained in the first of the further decomposition steps, the condensing gas mixture is subjected to heat exchange in the first condensation zone with at least part of the stripped urea synthesis solution which has been expanded to a pressure of 4-40 bar in the second decomposition step, and the non-condensed gas mixture from the first condensation zone is in part further condensed in a second condensation zone.

The process according to the invention is characterized in that the gas mixture used in a first evaporation step is formed by the gas phase resulting from the heat exchange between the part of the stripped urea synthesis solution expanded to a pressure of 4-40 bar and the condensing gas mixture in the first condensation zone. As a result, the heat released in the first condensation zone can be used as effectively as possible in the further process, first for the decomposition of the carbamate solution and subsequently for the evaporation of the urea synthesis solution. In this way the overall efficiency of the process can be improved.

The gas mixture used in the first evaporation step will preferably be made up by the gas phase obtained in expanding the stripped urea synthesis solution to a pressure of 4-40 bar. By first separating the gas and liquid phases upon expansion to a pressure of 4-40 bar, subsequently heating the obtained liquid phase in the first condensation zone and finally subjecting this latter part of the urea synthesis solution to a gas/liquid separation once more and feeding the two gas phases obtained to the first evaporation step, a higher efficiency is obtained in the heat exchange in the first condensation zone.

In a number of cases, in the first evaporation step heat will be supplied by condensation of steam, in addition to the heat obtained by condensation of the gas mixture obtained in the second decomposition step. In that case the amount of gas mixture from the first decomposition step which is passed directly to the second condensation zone will according to the invention be controlled such that a minimum amount of steam is required.

Dividing the gas mixture from the stripping zone provides the possibility of further optimizing the overall heat economy of the process, in the sense that the heat to be supplied via steam can be restricted further and may, particularly in the first evaporation step, be reduced to a means for rapidly controlling the temperature of the urea solution at the outlet of this evaporation step.

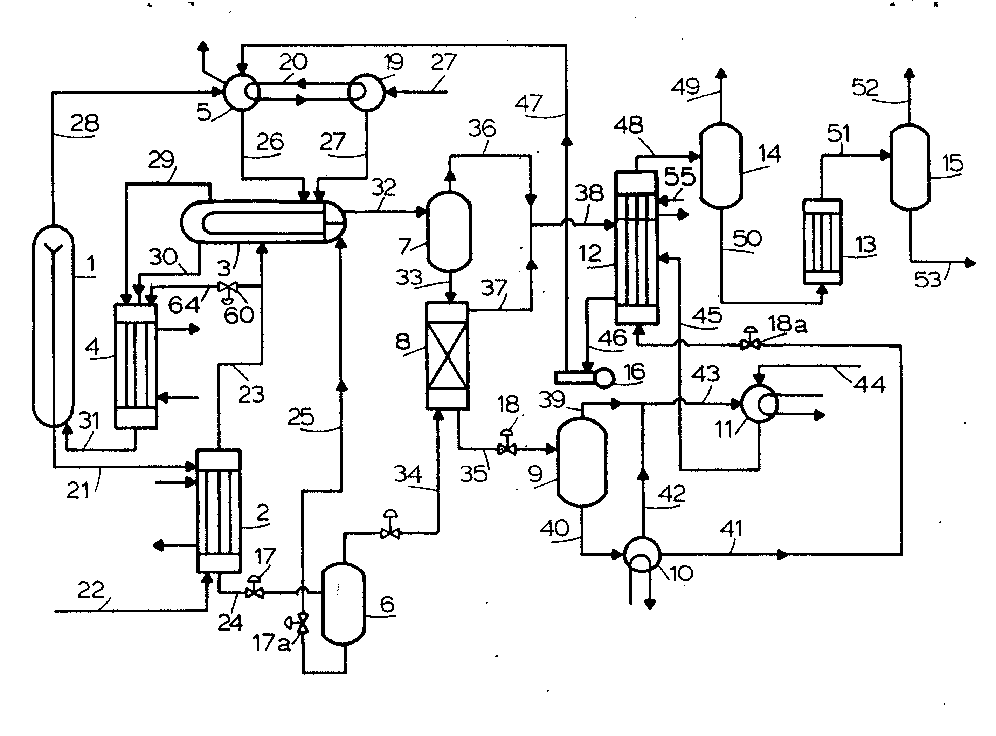

The invention will be elucidated with reference to the figure and the example without, however, being limited thereto.

The figure shows a synthesis zone indicated as 1, a stripping zone indicated as 2, a first and a second high pressure condensation zone as 3 and 4, respectively, and a scrubbing zone as 5. Liquid-gas separators are indicated as 6, 7 and 9. A zone for contacting liquids and gases is represented by 8. 10 represents a heater-decomposer and 11 a carbamate condensation zone operated at low pressure. The heating zones of a first and a second evaporation step are represented by 12 and 13, respectively, the devices for the separation of the water vapour formed in evaporation which belong to these heating zones are indicated by 14 and 15, respectively. 16 represents a carbamate pump; 17, 17a, 18 and 18a are expansion valves, 60 and 61 are control valves. The urea synthesis solution obtained in the urea synthesis zone 1 at a pressure of 125-250 bar, a temperature of 175-220¤C and a molar NH3/CO2 ratio of 2.7-4.0, for example 140 bar, 183¤C and a molar NH3/CO2 ratio of 2.95, which, in addition to urea and water, still contains free ammonia and unconverted ammonium carbamate, is fed, via 21, to the stripping zone 2. Carbon dioxide which, in a compression device not shown here, has been compressed to synthesis pressure and to which, if desired, passivating air has been added, is fed, via 22, to this stripping zone countercurrently to the urea synthesis solution. The stripping zone 2 is preferably designed as a vertical tubular heat exchanger. The heat required for stripping is obtained by condensation on the shell side of the heat exchanger of high pressure steam of, for example, 14-40 bar. The expelled gas mixture, which, in addition to ammonia and carbon dioxide, contains equilibrium amounts of water vapour, is passed, together with the carbon dioxide required for stripping, via 23, into the first condensation zone 3 and via line 64 and valve 60 into the second condensation zone 4. Condensation zone 3 is shown in the figure as a horizontal submerged condenser. In this zone the gas mixture is in part condensed to a carbamate-containing solution. A diluted carbamate solution, obtained by scrubbing ammonia and carbon dioxide in scrubbing zone 5 from the gas mixture containing inert gases and discharged, via 28, from the synthesis zone 1, is fed, via 26, to the first condensation zone 3. The heat developed during the formation of this diluted carbamate solution is used to heat the fresh liquid ammonia supplied via 27 or a different process flow. A heat exchanger 19 may be installed for this purpose in which the heat released in scrubbing zone 5 is transferred via a circuit 20. The residence time of the reaction mixture in the first condensation zone 3 is chosen such that in this zone in addition at least 30% of the equilibrium amount of urea that can be formed under the given process conditions, for example 20% by weight, is formed from carbamate. This raises the condensation temperature and a larger amount of usable heat becomes available This heat can be used to decompose further amounts of carbamate still present in the stripped urea synthesis solution. For this purpose the solution discharged, via 24, from the stripping zone 2 is expanded with the help of expansion valve 17 to a pressure of 4-40 bar, preferably 12-30 bar, for example 29.5 bar, and the resulting mixture is led into the gas-liquid separator 6. The liquid phase thus obtained, substantially an aqueous urea solution which also contains ammonia and carbon dioxide, is discharged via 25 and the gas phase, a mixture containing substantially ammonia, carbon dioxide and water vapour, via 34. The liquid phase discharged via 25 from the gas-liquid separator 6 is subsequently, at a pressure equal to or lower than that at which the expansion took place, for example 18.5 bar, via expansion valve 17a, brought into heat exchange with the solution containing carbamate and urea which is formed in the first condensation zone 3, during which process further amounts of carbamate present in the expanded stripped urea synthesis solution are decomposed into ammonia and carbon dioxide. The non-condensed part of the gas mixture supplied to the first condensation zone 3 is discharged from this zone via 29 and the solution containing carbamate and urea formed in this zone is carried off via 30 and introduced into the second high pressure condensation zone 4. In this zone the gas mixture supplied via 29 is further condensed to a carbamate solution. The heat released during this condensation process is carried off by means of water, which is thus converted into low pressure steam of 4-9 bar. The solution containing carbamate and urea obtained in this second high pressure carbamate condensation zone 4 and the non-condensed part of the supplied gas mixture containing ammonia, carbon dioxide and water vapour are introduced into synthesis zone 1 via 31.

The gas-liquid mixture obtained in the heat exchange in the first high pressure condensation zone 3 and discharged via 32 is fed into gas-liquid separator 7, from where the resulting gas phase, a gas mixture containing ammonia, carbon dioxide and water vapour, is discharged via 36 and the resulting liquid phase, a carbamate-containing urea solution, via 33. In the embodiment shown in the figure the liquid phase obtained via 33 is brought into contact, in contact zone 8, with the gas phase discharged, via 34, from gas-liquid separator 6, the excess of ammonia present in the liquid phase being expelled by the gas mixture rich in carbon dioxide, as a result of which a liquid phase is obtained, via 35, from the contact zone which is relatively less rich in ammonia. The urea-containing solution, still containing carbamate, that is removed from contact zone 8 is fed, via 35 and expansion valve 18, in which the pressure of the solution is lowered to 1-10 bar, for example 5 bar, to the gas-liquid separator 9. A urea-containing solution is discharged from here via 40. Carbamate still present in this solution is decomposed in heater-decomposer 10, which is heated with low pressure steam, upon which the urea solution is supplied, via 41 and expansion valve 18a, to heating zone 12 of the first evaporation step. Heating zone 12 may, for instance, be designed as a vertical tubular heat exchanger. The urea solution to be concentrated is passed through the tubes.

The gas phase obtained in gas-liquid separator 9, a gas mixture containing ammonia, carbon dioxide and water vapour, is combined with the gas mixture containing ammonia, carbon dioxide and water vapour obtained in the heater-decomposer 10 and discharged via 42, upon which the combined gas mixtures are passed, via 43, to the low pressure condensation zone 11 where they are condensed with an aqueous solution, for example process condensate, supplied via 44. The molar $NH_3/CO_2$ ratio in the combined gas mixtures is maintained between 2.0 and 4.5, for example at 4.1, so that the condensation can take place under optimum conditions. The carbamate solution obtained in the low pressure condensation zone 11 is fed, via 45, to the shell side of the heating zone 12. The gas mixture containing ammonia, carbon dioxide and water vapour obtained by combining flows 36 and 37 is also supplied to this shell side via 38. In 12 the condensing gas mixtures flow countercurrently to the urea solution to be evaporated. During the condensation of the gas mixture supplied via 38 with the help of the carbamate solution supplied via 45, sufficient heat is released to cover the heat requirements of the first evaporation step, in which the urea solution supplied via 41, which contains 70-75 wt% urea, is concentrated to a urea content of 85-95 wt%.

The top part of the heating zone 12 consists of a steam heating zone. Via 55, steam is supplied to the shell side of this heating zone, which enables the temperature of the concentrated urea solution discharged from 12 to be controlled. The heat content of the gas mixture supplied via 38 is further controlled by valve 60, which is to control the division between the two condensation zones 3 and 4 of the stripping gas supplied via 23. The carbamate solution formed in the condensation of the gas mixture on the shell side of the heat exchanger of the first concentration step 12 is discharged via 46, brought to synthesis pressure by means of carbamate pump 16 and fed into scrubbing zone 5 via 47. The water vapour from the mixture of concentrated urea solution and water vapour discharged via 48 from heating zone 12 of the first evaporation step is discharged, via 49, in the water vapour separator 14, and the concentrated urea solution is fed, via 50, to heating zone 13 of the second evaporation step 13. The vapour-liquid mixture formed here is passed, via 51, to the water vapour separator 15, from where the water vapour is carried off via 52 and the urea melt which is practically free of water via 53.

The invention is, however, not limited to the above-described embodiment. It is, for example, possible to omit contact zone 8 and to supply, via 34, the gas phase resulting from 6 directly to 37, while the liquids coming from 7 can be returned to 9 more directly via 35. It is even possible to omit the contact zone 8 as well as the gas-liquid separator 6 and to pass the entire urea synthesis solution obtained from stripper 2 upon expansion via valve 17, to the first condensation of 3.

The invention will now be further elucidated with reference to a few examples.

## Example 1

With the help of the method described, urea is prepared according to the embodiment as shown in the figure in an installation with three decomposition steps with a production capacity of 1500 tonnes a day. The amounts are given in kg per hour. The pressure applied in the high pressure part of the installation is 139 bar, upon expan sion in the second decomposition step 17.7 bar and in the final decomposition step 3.9 bar.

35,411 kg of $NH_3$ of 104¤C and 68,681 kg of a carbamate solution with a temperature of 165¤C, which contains 28,045 kg of $CO_2$, 26,356 kg of $NH_3$ and $H_2O$ are supplied to high pressure condensation zone 3. The temperature in the reaction zone 1 is 183¤C and the molar $NH_3/CO_2$ ratio of the urea solution is 2.95. 194,863 kg of urea synthesis solution is supplied to stripping zone 2 and then stripped with supply of heat with 45,863 kg of $CO_2$. A solution containing 63,528 kg of urea, 19,637 kg of $CO_2$, 17,258 kg of $NH_3$ and 33,170 kg of $H_2O$ is discharged from the stripping zone via 24 and a gas mixture consisting of 62,557 kg of $CO_2$, 41,432 kg of $NH_3$ and 3,219 kg of $H_2O$ via 23. The pressure of the stripped urea synthesis solution is subsequently lowered to 17.7 bar. As a result, in gas-liquid separator 6 8,566 kg of a gas mixture containing 6,238 kg of $CO_2$, 1,839 kg of $NH_3$ and 457 kg of $H_2O$ is obtained, which is discharged via 34. In addition, 125,267 kg of a liquid phase containing 63,528 kg urea, 13,398 kg of $CO_2$, 15,418 kg of $NH_3$ and 32,713 kg of $H_2O$ remain.

Of the gas mixture discharged from the stripping zone via 23, 74,109 kg is passed to high pressure condensation zone 3 and 34,828 kg to high pressure condensation zone 4.

The residence time of the mixture in zone 3 is chosen such that 20,021 kg of urea is also formed in this solution and that, besides this, the solution contains 40,473 kg of $CO_2$, 50,731 kg of $NH_3$ and 21,386 kg of $H_2O$. A further part of the non-condensed gas mixture discharged via 29 is then condensed in the second high pressure condensation zone 4, together with the gas mixture supplied via 64.

A solution containing 42,291 kg of urea. 48,872 kg of $CO_2$, 63,566 kg of $NH_3$ and 29,450 kg of $H_2O$ and a gas mixture consisting of 10,732 kg of $CO_2$, 15,681 kg of $NH_3$ and 723 kg of $H_2O$ are supplied to the synthesis zone.

In the first high pressure condensation zone 3 the released heat is carried off by means of liquid flow 25, in which process further decomposition into $NH_3$ and $CO_2$ of the carbamate present in this flow takes place. Upon gas-liquid separation of the resulting reaction mixture, a gas flow is obtained via 36, at a pressure of 17.7 bar and a temperature of 135¤C, which is mixed with the gas coming from contact zone 8 via 37. The resulting gas flow in 38 contains 16,225 kg of $CO_2$, 10,459 kg of $NH_3$ and 3,932 kg of $H_2O$ and has a temperature of 158¤C and via 35 a solution is obtained which, in addition to 63,528 kg of urea, contains 3,412 kg of $CO_2$, 6.788 kg of $NH_3$ and 29,238 kg of $H_2O$.

This urea-containing solution is further processed at a pressure of 3.3 bar to decompose the carbamate still present in the solution. Via 43 a gas mixture is thus obtained that contains 1176 kg of $CO_2$, 3193 kg of $NH_3$ and 1589 kg of $H_2O$. The urea solution obtained in 10, consisting of 63,528 kg of urea, 2,236 kg of $CO_2$, 3,604 kg of $NH_3$ and 27,649 kg of $H_2O$ with a temperature of 123¤C, is passed, via 41 and expansion valve 18a, to the heating zone 12 of the first evaporation step. During the expansion in expansion valve 18a the temperature drops to 85¤C. The heat required for concentration is obtained by condensing the combined gas flows 36 and 37 countercurrently to the urea solution on the shell side of the heating zone 12, with the help of the carbamate solution containing 4,740 kg of $CO_2$, 7,683 kg of $NH_3$ and 9,743 kg of water and having a temperature of 49¤C, obtained in the low pressure step and supplied via 45.

The carbamate solution leaving evaporator 12 via 46 contains, at a temperature of 113¤C, 20,966 kg of $CO_2$, 18,143 kg of $NH_3$ and 13,675 of kg $H_2O$ and is recycled via pump 16.

In addition, approx. 94 kg of steam of 145¤C and 4 bar was used for evaporation, which was thereby cooled to 143¤C.

## Example 2

Under otherwise equal conditions, but with a different position of valve 60, the following results were obtained. 35,414 kg of $NH_3$ of 104¤C and 65,638 kg of a carbamate solution with a temperature of 165¤C, containing 26,737 kg of $CO_2$, 25,318 kg of $NH_3$ and $H_2O$, are supplied to the high pressure condensation zone 3.

193,688 kg of urea synthesis solution is supplied to stripping zone 2, which solution is then stripped, with supply of heat, with 45,863 kg of $CO_2$. A solution containing 63,526 kg of urea, 18,405 kg of $CO_2$, 16,187 kg of $NH_3$ and 32,477 kg of $H_2O$ is discharged from the stripping zone via 24, and via 23 a gas mixture consisting of 63,548 kg of $CO_2$, 42,228 kg of $NH_3$ and 3,225 kg of $H_2O$. As a result, in the gas-liquid separator 6 8,566 kg of a gas mixture that contains 6,159 kg of $CO_2$, 1,799 kg of $NH_3$ and 466 kg of $H_2O$ is obtained, which is discharged via 34. In addition, 122,380 kg of a liquid phase remain, containing 63,526 kg of urea, 12,246 kg of $CO_2$, 14,387 kg of $NH_3$ and 32,010 kg of $H_2O$.

Of the gas mixture discharged from the stripping zone via 23, 58,471 kg is passed to high pressure condensation zone 3 and 52,288 kg to high pressure condensation zone 4. The residence time of the mixture in zone 3 is chosen such that 17,069 kg of urea is also formed in this solution and that the solution also contains 38,635 kg of $CO_2$, 50,704 kg of $NH_3$ and 19,627 kg of $H_2O$. A further part of the non-condensed gas mixture discharged via 29 is then condensed in the second high pressure condensation zone 4, together with the gas mixture supplied via 64. .

A solution containing 42,207 kg of urea, 48.674 kg of $CO_2$, 63,224 kg of $NH_3$ and 28,861 kg of $H_2O$ and a gas mixture containing 10,675 kg of $CO_2$, 15,831 kg of $NH_3$ and 716 kg of $H_2O$ are supplied to the synthesis zone. The gas flow in 38 contains 15,037 kg of $CO_2$, 9,471 kg of $NH_3$ and 3,568 kg of $H_2O$ and has a temperature of 158¤C, and via 35 a solution is obtained which, in addition to 63,526 63,528 kg of urea, contains 3,367 kg of $CO_2$, 6,715 kg of $NH_3$ and 28,906 kg of $H_2O$.

Via 43 a gas mixture containing 1,167 kg of $CO_2$, 3,163 kg $NH_3$ and 1,575 kg of $H_2O$ is obtained. The urea solution obained in 10 consists of 63,526 kg of urea, 2,200 kg of $CO_2$, 3,551 kg of $NH_3$ and 27,331 kg of $H_2O$ that has a temperature of 123¤C.

A carbamate solution containing 4,395 kg of $CO_2$, 7,487 kg of $NH_3$ and 3,393 kg of water and having a temperature of 47¤C is supplied via 45.

The carbamate solution leaving evaporator 12 via 46 contains, at a temperature of 103¤C, 19,432 kg of $CO_2$, 16,958 kg of $NH_3$ and 12,969 kg of $H_2O$. In addition, 995 kg of steam of 145¤C and 4 bar is used for evaporation, as a result of which the steam drops in temperature to 143¤C.

## Example 3

Under otherwise equal conditions, but with a different position of valve 60, the following results were obtained. 35,414 kg of $NH_3$ with a temperature of 96¤C and 63,426 kg of a carbamate solution with a temperature of 165¤C, containing 25,761 kg of $CO_2$, 24,522 kg of $NH_3$ and $H_2O$, are supplied to high pressure condensation zone 3. 193,087 kg of urea synthesis solution is supplied to stripping zone 2, which solution is stripped with 45,863 of kg $CO_2$ while heat is being supplied. A solution containing 63,525 kg of urea, 17,477 kg of $CO_2$, 15,367 kg of $NH_3$ and 32,040 kg of $H_2O$ is discharged from the stripping zone via 24 and a gas mixture consisting of 64,383 kg of $CO_2$, 42,941 kg of $NH_2$ and 3,290 kg of $H_2O$ via 23. In the gas-liquid separator 6 8,356 kg of a gas mixture containing 6,092 kg of $CO_2$, 1,759 kg of $NH_3$ and 475 kg of $H_2O$ is thus obtained, which is discharged via 34. In addition to this, 120.293 kg of a liquid phase remains, which contains 63,525 kg of urea, 11,384 kg of $CO_2$, 13,607 kg of $NH_3$ and 31,565 kg of $H_2O$.

Of the gas mixture discharged from the stripping zone via 23, 51,272 kg is supplied to high pressure condensation zone 3 and 61,072 kg to high pressure condensation zone 4.

The residence time of the mixture in zone 3 is chosen such that 14,978 kg of urea is also formed in this solution and that, in addition, the solution contains 37,385 kg of $CO_2$, 50,500 kg of $NH_3$ and 18,464 kg of $H_2O$.

A further part of the non-condensed gas mixture discharged via 29 is then condensed, together with the gas mixture supplied via 64, in the second high pressure zone 4.

A solution containing 43,461 kg of urea, 47,822 kg of $CO_2$, 62,722 kg of $NH_3$ and 28,841 kg of $H_2O$ and a gas mixture containing 10,466 kg of $CO_2$, 15,539 kg of $NH_3$ and 708 kg of $H_2O$ are supplied to the synthesis zone.

The resulting gas flow in 38 contains 14,138 kg of $CO_2$, 8,710 kg of $NH_3$ and 3,301 kg of $H_2O$ and has a temperature of 158¤C, and via 35 a solution is obtained which, besides 63,525 kg of urea, contains 3,337 kg of $CO_2$, 6,656 kg of $NH_3$ and 28,739 kg of $H_2O$. Via 43 a gas mixture is obtained which contains 4,293 kg of $CO_2$, 7,400 kg of $NH_3$ and 2,228 kg of $H_2O$. The urea solution obtained in 10 consists of 63,525 kg of urea, 2,175 kg of $CO_2$, 3,516 kg of $NH_3$ and 27,171 kg of $H_2O$ with a temperature of 123¤C.

A carbamate solution containing 4,293 kg of $CO_2$, 7,400 kg of $NH_3$ and 9,228 kg of water and having a temperature of 49¤C is supplied via 45.

The carbamate solution leaving evaporator 12 via 46 contains 18,432 kg of $CO_2$, 16,121 kg of $NH_3$ and 12,530 kg of $H_2O$ at a temperature of 93¤C. In addition, 1,818 kg of steam of 145¤C and 4 bar was used in evaporation, which is thereby cooled to 143¤C. The examples clearly show that, depending on the distribution of the gas phase supplied via 23 between the condensation zones, it is possible to control the amount of steam to be supplied in the first evaporation step.

## Claims

1. Process for preparing urea in which,
-in a synthesis zone, at a pressure of 125-350 bar and the corresponding temperature, a urea synthesis solution containing carbamate and free ammonia is formed from carbon dioxide and an excess of ammonia,
-in a first decomposition step part of the carbamate is decomposed, at synthesis pressure or lower, with supply of heat and in countercurrent contact with a stripping gas, and the resulting gas phase is in part condensed in a first condensation zone and the condensate and the non-condensed part of the gas mixture are returned to the synthesis zone,
-in at least two further decomposition steps a further part of the carbamate still present is decomposed and the gas mixture formed is separated off, in the first of the further decomposition steps a pressure of 4-40 bar being maintained and heat being supplied,
-the remaining urea-containing solution is processed by evaporation to form a concentrated urea solution, the heat required for evaporation being obtained at least in part by condensation, at the pressure of 4-40 bar, of the gas mixture obtained in the first of the further decomposition steps, the condensing gas mixture is subjected to heat exchange in the first condensation zone with at least part of the stripped urea synthesis solution, which has been expanded to a pressure of 4-40 bar in the second decomposition step, and the non-condensed gas mixture from the first condensation zone is, in part, further condensed in a second condensation zone,
characterized in that the gas mixture used in the first evaporation step is formed by the gas phase resulting from the heat exchange between the part of the stripped urea synthesis solution expanded to a pressure of 4-40 bar and the condensing gas mixture in the first condensation zone.

2. Process according to claim 1, characterized in that the gas mixture used in the first evaporation step is made up with the gas phase obtained in expanding the stripped urea synthesis solution to a pressure of 4-40 bar.

3. Process according to claim 1 or 2, characterized in that part of the gas mixture formed in the first decomposition step is passed directly to the second condensation zone.

4. Process according to any one of claims 1-3, characterized in that in the first evaporation step, in addition to the heat obtained by condensation of the gas mixture obtained in the second decomposition step, heat is also supplied by condensation of steam, and the amount of gas mixture from the first decomposition step which is passed directly to the second condensation zone is controlled in such a manner that a minimum amount of steam is required.

5. Process according to claim 4, characterized in that the amount of steam required in the first evaporation step is controlled by controlling the amount of gas mixture formed in the first decomposition step which is passed directly to the second condensation step.

6. Process for preparing urea as described and elucidated by means of the figures.

7. Urea and urea solutions prepared by applying the process according to one or more of the preceding claims.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 145 054 (UNIE VAN KUNSTMESTFABRIEKEN B.V.) * Whole document * | 1 | C 07 C 126/02 C 07 C 126/08 |
| D,A | GB-A-2 083 472 (MITSUI TOATSU CHEMICALS) * Complete specification * | 1 | |
| D,A | EUROPEAN CHEMICAL NEWS UREA SUPPLEMENT, 17th January 1969, pages 18-20: "DSM carbon dioxide stripping process" * Pages 18-20 * | 1 | |

TECHNICAL FIELDS SEARCHED (Int. Cl.4)

C 07 C 126/00

The present search report has been drawn up for all claims

| Place of search | Date of completion of the search | Examiner |
|---|---|---|
| THE HAGUE | 14-12-1987 | MAISONNEUVE J.A. |

EPO FORM 1503 03.82 (P0401)